# EUROPÄISCHE PATENTSCHRIFT

(11) **EP 2 976 116 B1**
(45) Veröffentlichungstag und Bekanntmachung des Hinweises auf die Patenterteilung: **19.07.2017**
(21) Anmeldenummer: 14713415.9
(22) Anmeldetag: 19.03.2014
(51) Int. Cl.: A61M 5/145

(54) **VORRICHTUNG UND VERFAHREN ZUR AUFNAHME EINER SPRITZE IN EINE FLUIDABGABEVORRICHTUNG**
APPARATUS AND METHOD FOR RECEIVING A SYRINGE IN A FLUID ADMINISTRATION DEVICE
APPAREIL ET PROCEDE POUR RECEVOIR UNE SERINGUE DANS UN DISPOSITIF D'ADMINISTRATION DE FLUIDE

(30) Priorität: 21.03.2013 DE 102013004860
(43) Veröffentlichungstag der Anmeldung: 27.01.2016
(73) Patentinhaber: Fresenius Medical Care Deutschland GmbH, 61352 Bad Homburg (DE)
(72) Erfinder: FAULHABER, Thomas, 97493 Bergrheinfeld (DE); WEINGÄRTNER, Thomas, 97218 Gerbrunn (DE)
(74) Vertreter: Ziermann, Oliver
(86) Internationale Anmeldenummer: PCT/EP2014/055550
(87) Internationale Veröffentlichungsnummer: WO 2014/147150

(56) Entgegenhaltungen:
- EP-A1- 1 588 729
- WO-A1-01/08726
- US-A1- 2012 195 793
- US-B2- 8 070 732

## Beschreibung

Die Erfindung bezieht sich auf eine Vorrichtung zur Aufnahme einer Spritze in eine Fluidabgabevorrichtung sowie ein Verfahren hierzu. Die Erfindung bezieht sich ferner auf eine Behandlungseinheit mit einer Vorrichtung nach dem Oberbegriff des Anspruch 1. Darüber hinaus betrifft die Erfindung die Verwendung einer Vorrichtung zur Aufnahme einer Spritze in eine Fluidabgabevorrichtung. Die Vorrichtung kann in vielen Bereichen Anwendung finden, wie z. B. im technischen, analytischen, pharmazeutischen oder medizinischen Bereich.

Es ist bekannt, zur Förderung genau bemessener Flüssigkeitsmengen Dosiervorrichtungen in Form von sogenannten Dosierpumpen einzusetzen. Diese liefern unabhängig von den Druckverhältnissen am Eingang und Ausgang der Pumpe definierte Volumina pro Hub und Zeit.

Dosierpumpen kommen insbesondere in der Medizin zur Anwendung, da hier die genaue Dosierung von Medikamenten und die Konstanterhaltung der Wirkstoffkonzentration von besonderer Bedeutung ist.

Die in der Medizin zur Dosierung von Arzneimitteln bei der kontinuierlichen intravenösen Verabreichung eingesetzten Dosierpumpen werden als Infusionspumpen bezeichnet.

Ebenfalls in der Medizin eingesetzt werden sogenannte Spritzenpumpen. Darunter versteht man eine Dosierpumpe zur intravenösen Verabreichung von Medikamenten, die sich in Spritzen befinden, welche in die dafür vorgesehene Aufnahme der Spritzenpumpe eingelegt werden.

Im besonderen Fall kann die Spritzenpumpe eine Heparinpumpe sein, die zusammen mit einem Dialysegerät zur genauen Dosierung von Medikamenten, die der Gerinnungshemmung (Antikoagulierung) des Blutes dienen, verwendet wird.

Eine Spritze besteht aus einem zylindrischen Hohlraum, dem Spritzenmantel, in dem ein beweglicher Kolben auf- und abgleiten kann. Am vorderen Ende wird der Spritzenmantel durch eine Deckplatte verschlossen, die in einem vorne offenen Konus, der Spritzendüse oder in einem Gewinde, beispielsweise einem LuerLock, ausläuft. An den Konus beziehungsweise LuerLock können eine Hohlnadel oder ein Schlauch angeschlossen werden. Am hinteren Ende des Spritzenmantels befindet sich in der Regel ein Kolbenstopp, um das Herausgeleiten des Spritzenkolbens zu verhindern.

Gängige Einwegspritzen bestehen aus Kunststoff. Mehrwegspritzen, die heute seltener eingesetzt werden, können aus Glas, Metall oder Gummi hergestellt sein.

Wie allgemein bekannt ist, weisen die Spritzen der verschiedenen Hersteller unterschiedliche Größen auf. Handelsübliche Spritzen erreichen beispielsweise ein Volumen von 0,5 ml bis 100 ml.

Selbst wenn Spritzen für die Aufnahme gleicher Flüssigkeitsmengen konstruiert sind, so können sich die äußeren Abmessungen der Spritze sowohl in der Länge als auch im Durchmesser von Hersteller zu Hersteller erheblich unterscheiden.

Dabei sind nicht nur Durchmesser und die Länge des Spritzenmantels sehr variabel sondern auch die Kolbenlängen sowie deren Durchmesser und insbesondere die Dicken des Kolbenflansches unterscheiden sich. Bei den handelsüblichen Spritzen variiert die Stärke des Kolbenflansches beispielsweise zwischen 1,2 mm und 3,0 mm.

Um die in eine Spritzenpumpe eingelegte Spritze zu entleeren, bewegt ein Antriebselement den Kolben der Spritze linear in dieser. Hierzu wird der Kolbenflansch von einer mit dem Antriebselement verbundenen Halterung umfasst. Dabei muss gewährleistet sein, dass der Kolben unterschiedlich großer Spritzen fest und sicher gehalten wird.

Aus dem Stand der Technik sind bereits zahlreiche Fluidabgabesysteme bekannt, die unterschiedliche Vorrichtungen zur Aufnahme und Halterung von Spritzen beinhalten.

So beschreibt das Dokument EP 1 847 285 ein Flüssigkeitsinjektionssystem mit einem Greifmechanismus aus einem Paar Klemmhaken, die sich einzeln zur Seite hin öffnen und schließen lassen, um einen linken und rechten Rand des Kolbenflansches zu erfassen. Sensoren und eine Auswerteinheit lassen erkennen, ob sich die Klemmhaken zunächst geöffnet und dann wieder geschlossen haben.

Die EP 0 514 907 beschreibt eine Vorrichtung zum Antreiben des Kolbens unterschiedlich großer Spritzen. Diese weist ein Paar bogenförmige Arme auf, die zueinander mittels Federkraft vorbelastet sind und eine Kraft in Richtung Mitte des Kolbenflansches ausüben. Eine weitere Klammer dient zur Halterung der Spritze in der Aufnahme.

Die EP 1 200 143 beschäftigt sich ebenfalls mit einem Antriebssystem für Fluidabgabevorrichtungen, welches unterschiedliche Spritzengrößen aufzunehmen vermag. Zwei schwenkbare, federbelastete Arme halten den Flansch des Tauchkolbens der Spritze von seiner inneren Seite aus an die Druckfläche des Antriebssystems gepresst und umfassen gleichzeitig klemmend den Schaft des Kolbens. Dadurch soll verhindert werden, dass der Spritzenkolben durch eine sogenannte "Siphonwirkung", d.h. einen Sog in die Spritze hineinbewegt und somit unbeabsichtigt Flüssigkeit aus der Spritze abgegeben wird.

Aus der EP 1 005 875 ist eine Flüssigkeitsinfusionsvorrichtung mit einer Haltevorrichtung bekannt, die den Flansch des Spritzenkolbens ebenfalls mechanisch zurückhält, um zu verhindern, dass ein negativer Druck an der in der Flüssigkeitsinfusionsvorrichtung angeordneten Spritze den Kolben in die Spritze zieht. Das genannte Dokument stellt eine Weiterentwicklung zum Dokument EP 0 916 353 dar.

Das europäische Patent EP 1 588 729 schlägt ein System zur Flüssigkeitsabgabe vor, welches zwei drehbare Klemmhaken mit gebogenen Enden aufweist. Eine Feder, die beide Haken miteinander verbindet, schließt die Haken nach dem Einlegen einer Spritze automatisch, wobei der Flansch des Kolbens durch eine Rotationsbewegung der Klemmhaken gefasst wird.

Aus der US 8,070,732 ist eine Spritzenpumpe mit einer Halterung bekannt, deren zwei über Zahnräder bewegbaren Greifarme nacheinander translatorisch und rotatorisch bewegt werden, um den Kolbenflansch zu greifen. Die Greifarme sind über Zahnräder miteinander verbunden.

Um Spritzen unabhängig von ihrer Größe aufnehmen zu können wird bei der Auslegung der Spritzenaufnahme meist ein relativ großes "Spiel" festgelegt.

Der Begriff "Spiel" bezeichnet den fertigungs- und anwendungsbedingten Bewegungsfreiraum, in dem sich ein mechanisches Bauteil nach der Montage gegen oder mit einem anderen Bauteil frei bewegen lässt. Im Normalfall ist ein großes Spiel vorteilhaft, beispielsweise im Hinblick auf problemlose Montage, Vermeidung von Verspannungen, Unempfindlichkeit gegen Temperaturschwankungen und Verschmutzung.

Bei der Anwendung von Spritzenpumpen birgt ein großes Spiel jedoch die Gefahr einer fehlerhaften Medikamentendosierung. Insbesondere bei der Dosierung im Unterdruckbereich kann es zu einem Wechsel der Anlagefläche zwischen Kolbenaufnahme und Spritzenkolben kommen. Bei jedem Wechsel wird das Spiel herausgedrückt, was sich als kleiner Dosierfehler bemerkbar macht.

Hier ist ein vernachlässigbar geringes Spiel zwischen den Teilen und insbesondere eine spielfreie Anbindung des Spritzenkolbens an die Antriebvorrichtung im Sinne einer Presspassung von Vorteil.

Die genannten Patentanmeldungen befassen sich mit dem Problem der Aufnahme unterschiedlicher Spritzengrößen. In welcher Art und Weise eine Spritzenaufnahme spielfrei erfolgen kann, wird jedoch nicht offenbart.

Der Erfindung liegt daher die Aufgabe zugrunde, eine Vorrichtung und ein Verfahren zur Verfügung zu stellen, die es ermöglichen, den Spritzenkolben unabhängig von der Größe der Spritze spielfrei aufzunehmen und ihn in dieser Position fest und sicher zu halten und somit die Nachteile der bisherigen Systeme auszuräumen.

Eine weitere Aufgabe der Erfindung ist die leichte Anwendbarkeit, d.h. das komfortable, leichte Einlegen der Spritze.

Darüber hinaus soll die Vorrichtung kostengünstig und einfach herstellbar sein.

Die Aufgabe wird erfindungsgemäß durch die Merkmale der Ansprüche 1 und 12 gelöst. Dabei besitzt die Vorrichtung zur Aufnahme der Spritze in einer Fluidabgabevorrichtung mindestens zwei federnd vorspannbare Klemmmittel, die den Kolbenflansch eines Spritzenkolbens greifen, wobei für jedes Klemmmittel ein Langloch vorgesehen ist, in dem das Klemmmittel gleitet und somit während des Greifvorgangs gleichzeitig eine rotatorische und translatorische Bewegung ausführt.
Vorteilhafte Ausführungsformen der Erfindung ergeben sich aus den Unteransprüchen.
Anspruch 16 betrifft eine medizintechnische Behandlungseinheit mit der erfindungsgemäßen Vorrichtung.
Die Verwendung der erfindungsgemäßen Vorrichtung, welche für den Einsatz im technischen, vorzugsweise analytischen, medizinischen und pharmazeutischen Bereich geeignet ist, wird durch Anspruch 17 umfasst.
Anspruch 18 betrifft eine Auswerteeinheit für die erfindungsgemäße Vorrichtung mit Sensoren, die die Position des Kolbenflansches und des Halteelements erkennen lassen.
Die erfindungsgemäße Vorrichtung besteht aus einer Halterung, mindestens zwei Klemmmitteln und einem Gehäuse, in dem die Klemmmittel gehaltert sind.
Das Gehäuse kann aus einer oberen und einer unteren Hälfte bestehen.
Die Halterung ist Bestandteil einer Dosiervorrichtung, die in einer bevorzugten Ausführungsform eine Spritzenpumpe darstellt, und steht mit einer Antriebsvorrichtung in Verbindung.
Die Dosiervorrichtung ist in einer besonderen Ausführungsform integraler Bestandteil einer Geräteplatte oder lösbar mit dieser über eine Befestigungsvorrichtung verbunden. Die Geräteplatte kann in bestimmten Ausführungsformen Teil einer medizinischen Behandlungsvorrichtung sein.

Die Antriebsvorrichtung vermag den Kolben einer Spritze zu bewegen, um das darin befindliche Fluid zu entleeren.

Eine Spritze besteht aus einem unterschiedlich langen und dicken zylindrischen Hohlraum, dem Spritzenmantel, in dem ein entsprechend großer beweglicher Kolben in axialer Richtung zur Spritze auf- und abgleiten kann.

Als Fluid werden allgemein Flüssigkeiten, Gase, Emulsionen oder Suspensionen bezeichnet. In bestimmten Ausführungsformen kann das Fluid ein medizinisches Fluid, wie beispielsweise Blut, Dialysat, Substituat, eine Spül-, Priming-, Sterilisations- oder Medikamentenlösung und dergleichen sein. In einer besonderen Ausführungsform ist das Fluid eine Medikamentenlösung.

Um die in eine Spritzenpumpe eingelegte Spritze zu entleeren, bewegt eine Antriebsvorrichtung den Kolben der Spritze linear zu dieser. Hierzu wird der Kolbenflansch mittels geeigneter Klemmmittel über die Halterung mit der Antriebsvorrichtung verbunden.

Die Antriebsvorrichtung basiert auf einem Elektromotor, aber auch ein federmechanischer und/oder pneumatischer und/oder magnetischer Betrieb ist denkbar.

Ein Klemmmittel besteht aus einem Betätigungselement und einem Halteelement. Beide Elemente können auch einstückig miteinander verbunden sein.

Das Betätigungselement wird durch den Benutzer betätigt, um das Haltelement, das den Kolbenflansch des Spritzenkolbens gefasst hält, von diesem zu trennen. Am Übergang des Betätigungselements in das Haltelement befindet sich eine Querstrebe, über die das Klemmmittel in dem Gehäuse gehaltert ist.

Zwei Abstandsräder an den beiden Enden der Querstrebe sorgen für einen genügend großen Abstand zwischen Betätigungs- bzw. Halteelement und Gehäuse, um eine reibungslose Bewegung zu ermöglichen.

An der Querstrebe sind zwei Vorsprünge ausgebildet, wobei der "erste" eine hakenähnliche und der "zweite" eine kurvenähnliche Form besitzt.

Der hakenähnliche Vorsprung steht mit einer Druckplatte in Verbindung, über die mittels mindestens einer Feder eine Vorspannung der Klemmmittel aufgebaut werden kann.

Die vorgespannte Feder hebt mindestens die Kraft des während des Betriebes in der Spritze entstehenden Unterdrucks auf und verhindert somit ein unterdruckbedingtes Einziehen des Kolbens in die Spritze und eine daraus resultierende unbeabsichtigte Flüssigkeitsabgabe.

Die hierzu mindestens (Fmin) und maximal notwendige Federkraft (Fmax) korreliert dabei mit der Dicke des Kolbenflansches.

Zur Bestimmung der Federkraft Fmin und Fmax werden der Unterdruck in der Spritze (pmin, pmax) und die Federspannung (Fnenn) in ein Verhältnis gesetzt. Die Federspannung ist dabei von der Auslegung der Feder, d.h. deren Länge, Material und Wicklung abhängig.

Die zur Betätigung des Klemmmittels erforderliche Kraft ergibt sich aus dem Verhältnis der verschiedenen Variablen Auslegung der Feder, Dicke des Kolbenflansches und Federkraft.

Bei Betätigung des Klemmmittels gleitet der kurvenähnliche Vorsprung aus einer Vertiefung auf eine Erhebung.

Der Winkel der Erhebung kann dabei vorzugsweise zwischen 0° und 50° betragen. Bei größerem Winkel erhöht sich die zur Betätigung der Klemmmittel notwendige Kraft.

Werden Federkraft und Winkel der Erhebung in geeigneter Weise aufeinander abgestimmt, können die Klemmmittel durch den Benutzer einhändig betätigt werden. So ist ein komfortables Einlegen oder Entnehmen der Spritze möglich.

Bei Betätigung des Klemmmittels dreht sich die Querstrebe einerseits um die eigene Achse und vollzieht andererseits das Herausgleiten aus der Vertiefung und Heraufgleiten auf die Erhebung in dem dafür vorgesehenen Langloch axial zum Kolben der Spritze nach, so dass gleichzeitig eine translatorische und rotatorische Bewegung stattfinden.

Durch die Verknüpfung dieser beiden Bewegungsformen ergreift das Haltelement den Kolbenflansch seitlich und von oben einschenkend und gewährleistet somit im Gegensatz zu einer reinen Kurvenbewegung eine spielfreie Halterung auch bei variierenden Kolbenflanschstärken von 1,2 mm bis 3,00 mm.

Das Halteelement gewährleistet zudem eine feste und sichere Halterung des Spritzenkolbens in einer exakten Position für die Dauer der Anwendung.

In einer besonderen Ausführungsform ist in das Gehäuse eine Auswerteeinheit mit mindestens einem Sensor integriert, über welche eine Aussage über die Position des Spritzenkolbens und/oder des Halteelements zu treffen ist. Dabei kann über die Bestimmung der Position des Spritzenkolbens beispielsweise das spielfreie Anliegen des Kolbenflansches am Gehäuse und über die Position des Halteelements dessen Öffnungsgrad bestimmt werden.

Sämtliche Elemente der Vorrichtung zur Aufnahme einer Spritze bestehen aus einem Material, das formstabil ist und einen geringen Verschleiß aufweist. In bestimmten Ausführungsformen kann das Material beispielsweise ein thermoplastischer Kunststoff oder ein Metall sein.

Die Verwendung eines frei erhältlichen Materials und die Reduktion auf wenige Bauteile ermöglichen eine kostengünstige und einfache Herstellung und gewährleisten, dass nur wenige Bauteile einem möglichen Verschleiß oder Defekt unterliegen. Somit kann auch der Wartungsaufwand für das Gesamtsystem gesenkt werden.

Die Vorrichtung zur Aufnahme einer Spritze ist in bestimmten Ausführungsformen als Einwegartikel bzw. "Disposable" denkbar, das nach Gebrauch, beispielsweise zusammen mit der Spritze, entsorgt wird.

Die beschriebene Vorrichtung kann in vielen Bereichen Anwendung finden, wie z.B. im technischen, analytischen, pharmazeutischen und medizinischen Bereich.

Im Folgenden wird die Erfindung unter Bezugnahme auf die Zeichnungen näher erläutert.

Es zeigen
- **Figur 1**: eine dreidimensionale Darstellung einer Fluidabgabevorrichtung mit der erfindungsgemäßen Aufnahmevorrichtung;
- **Figur 2**: eine dreidimensionale Darstellung des Klemmmittels;
- **Figur 3**: eine dreidimensionale Darstellung einer Ausführungsform der erfindungsgemäßen Vorrichtung mit geöffneten Klemmmitteln mit dem Gehäuse im Längsschnitt;
- **Figur 4**: eine schematische Darstellung einer Ausführungsform der erfindungsgemäßen Vorrichtung mit geöffneten Klemmmitteln im Längsschnitt;
- **Figur 5**: eine dreidimensionale Darstellung einer Ausführungsform der erfindungsgemäßen Vorrichtung mit geschlossenen Klemmmitteln mit dem Gehäuse im Längsschnitt;
- **Figur 6**: eine schematische Darstellung einer Ausführungsform der erfindungsgemäßen Vorrichtung mit geöffneten Klemmmitteln im Längsschnitt;
- **Figur 7**: eine dreidimensionale Darstellung des Klemmmittels.

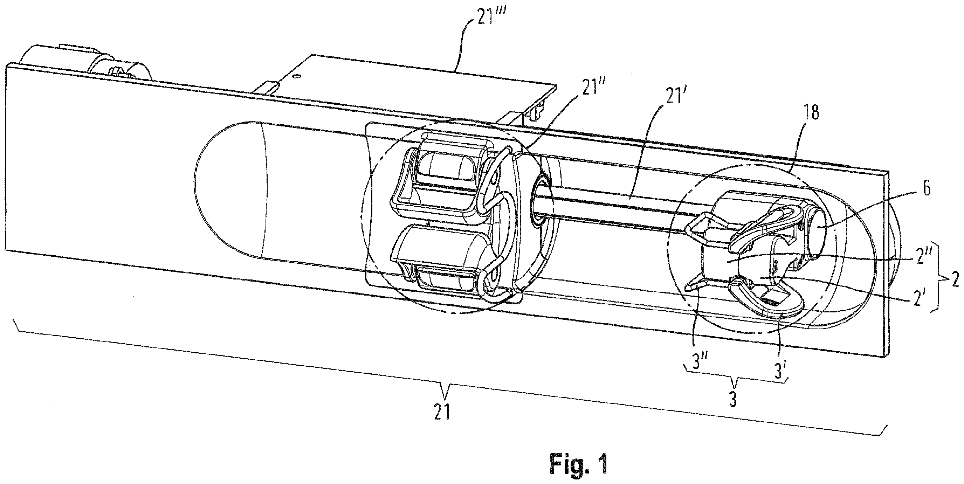

**Figur 1** zeigt eine Fluidabgabevorrichtung 21 mit der erfindungsgemäßen Aufnahmevorrichtung 18 in dreidimensionaler Darstellung, die aus einem formstabilen Material, vorzugsweise Kunststoff oder Metall, bestehen kann. Die Aufnahmevorrichtung 18 ist über eine Öffnung 5 mit einer Gleitstange 21' verbunden. Letztere kann über eine Antriebsvorrichtung bewegt werden. Der Spritzenmantel wird in die Spritzenhalterung 21" eingelegt. Über eine Befestigungsvorrichtung 21"' kann die Fluidabgabevorrichtung 21 an ein medizintechnisches Gerät konnektiert werden.

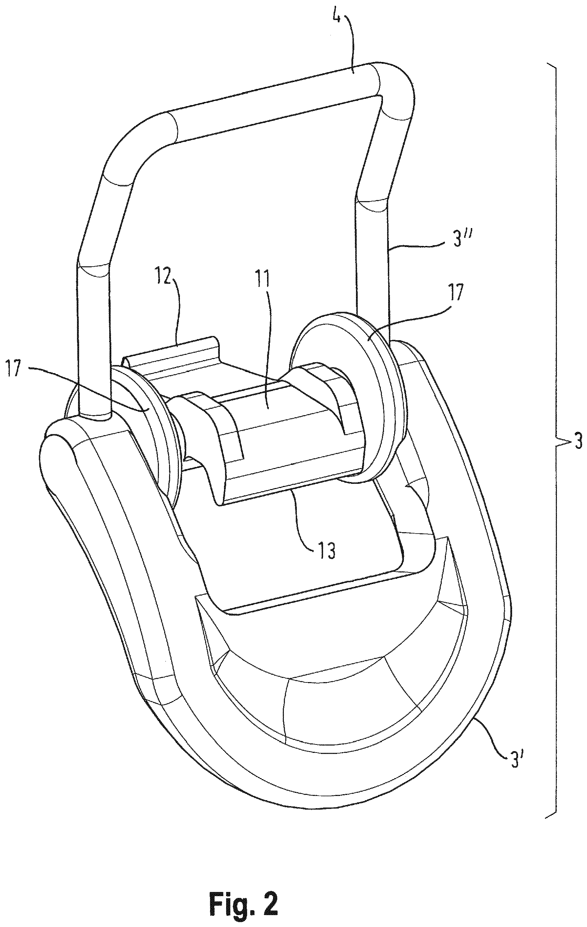

**Figur 2** zeigt eine schematische dreidimensionale Darstellung des Klemmmittels 3. Betätigungselement 3' und Halteelement 3" sind über eine Querstrebe verbunden. An dieser befinden sich ein hakenähnlicher 12 und ein kurvenähnlicher Vorsprung 13. Der hakenähnliche Vorsprung 12 der Querstrebe 11 steht mit einer Druckplatte 8 in Verbindung, über die die Klemmmittel 3 mittels mindestens einer Feder 7 vorgespannt werden können. Der kurvenähnliche Vorsprung 13 der Querstrebe 11 liegt in seiner Ausgangsposition, d.h. bei geöffneter Klammer, in einer Vertiefung 14. Betätigungs- und Halteelement können auch einstückig ausgeführt sein.

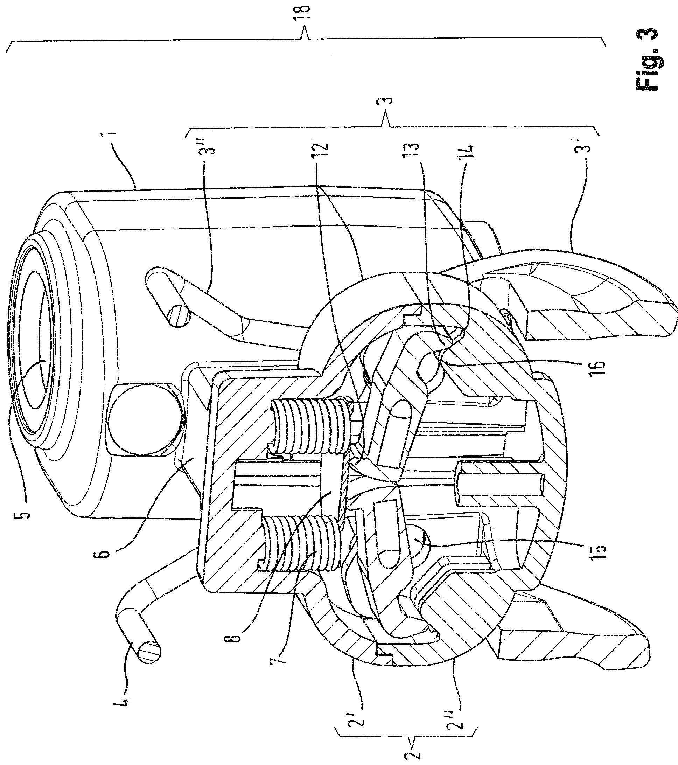

**Figur 3** zeigt eine Ausführungsform der erfindungsgemäßen Vorrichtung in dreidimensionaler Darstellung mit geöffneten Klemmmitteln 3 bei längsgeschnittenem Gehäuse 2.

Die erfindungsgemäße Aufnahmevorrichtung 18 weist ein an einer mit einer Öffnung für eine Gleitstange 5 versehenen Halterung 1 befestigtes Gehäuse 2 auf. Das Gehäuse besteht aus einer oberen Hälfte 2' und einer unteren Hälfte 2". Denkbar ist auch eine einstückige Ausführung des Gehäuses 2.

In dem Gehäuse 2 sind über jeweils eine Querstrebe 11 zwei Klemmmittel 3 in einem Langloch 15 gehaltert.

Die Klemmmittel 3 bestehen ihrerseits aus einem Betätigungselement 3' und einem Halteelement 3". Das Halteelement weist ein klammerähnliches Ende 4 auf und greift damit den Kolbenflansch. Die Stärke des Kolbenflansches kann 1,2 mm bis 3,0 mm betragen. Die Querstrebe 11 befindet sich am Übergang zwischen Betätigungselement 3' und Haltelement 3" und weist ihrerseits an den Enden jeweils ein Abstandsrad 17 auf. Das Abstandsrad 17 sorgt für einen genügend großen Abstand zum Gehäuse 2 um eine reibungslose Bewegung zu ermöglichen.

In einer Ausführungsform ist die Aufnahmevorrichtung integraler Bestandteil einer medizintechnischen Behandlungseinheit oder über eine Befestigungsvorrichtung 21"' lösbar mit dieser verbunden und dient der Fluidverabreichung.

Die Aufnahmevorrichtung kann auch in eine Fluidabgabevorrichtung, vorzugsweise eine Dosiervorrichtung und insbesondere eine Spritzenpumpe integriert oder lösbar mit dieser verbunden sein.

In einer besonders bevorzugten Ausführungsform ist die Aufnahmevorrichtung integraler Bestandteil einer Heparinpumpe oder lösbar mit dieser verbunden.

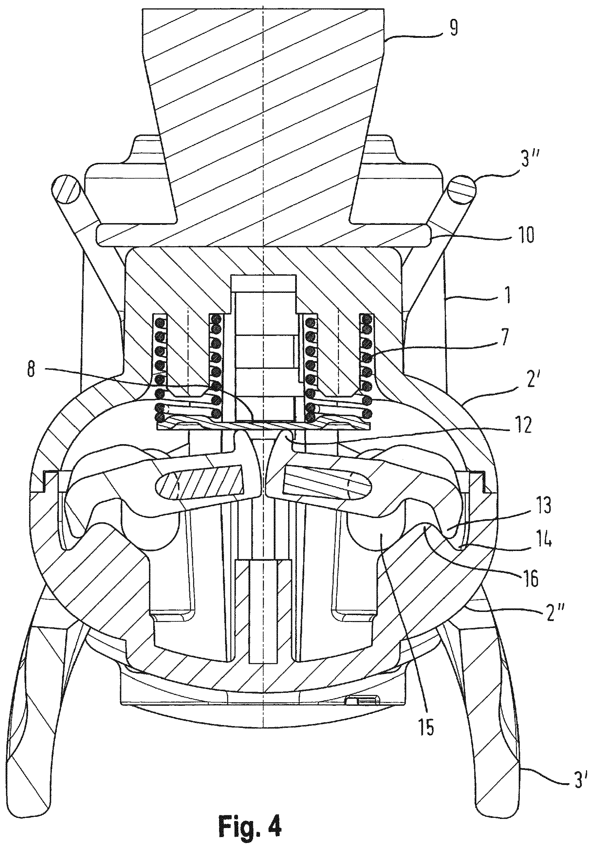

**Figur 4** zeigt in schematischer Darstellung eine Ausführungsform der erfindungsgemäßen Aufnahmevorrichtung 1 mit geöffneten Klemmmitteln im Längsschnitt.

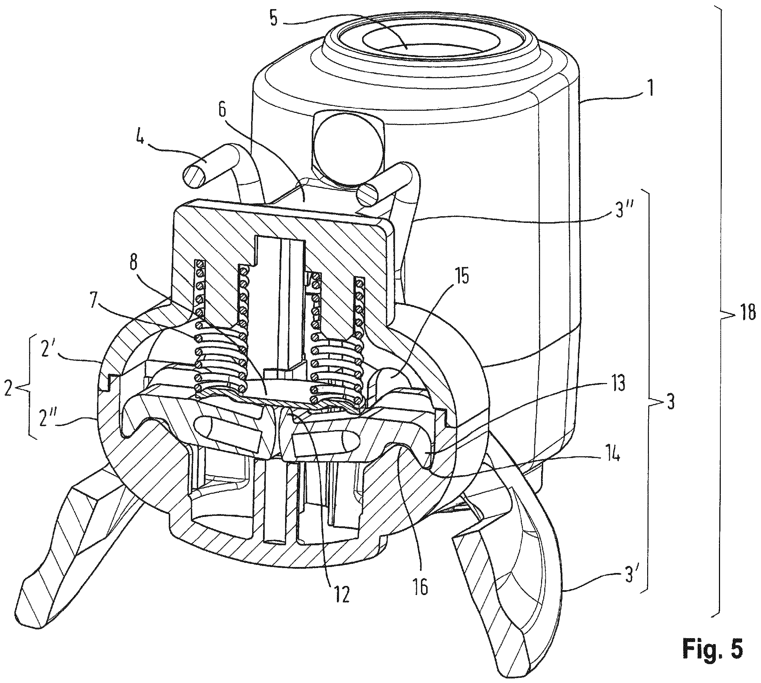

**Figur 5** zeigt eine Ausführungsform der erfindungsgemäßen Vorrichtung in dreidimensionaler Darstellung mit geschlossenen Klemmmitteln 3 bei längsgeschnittenem Gehäuse 2.

Zum Einlegen des Spritzenkolbens 9 in die Aufnahmevorrichtung 18 muss das Betätigungselement 3' des Klemmmittels 3 aktiviert werden.

Der kurvenähnliche Vorsprung 13 gleitet dabei aus der Vertiefung 14 auf die Erhebung 16. Dadurch bewegt sich die Querstrebe 11 axial zur Spritze im Langloch 15 nach oben in Richtung Spritzenkolben 9 (translatorische Bewegung) und dreht sich gleichzeitig um die eigene Achse (rotatorische Bewegung). Der Winkel der Erhebung kann dabei vorzugsweise zwischen 0° und 50° betragen. Der hakenähnliche Vorsprung 12 der Querstrebe 11 bewegt die Druckplatte 8 nach oben in Richtung Spritzenkolben und presst die Feder 7 zusammen. Das Haltelement 3" ist nun abgespreizt.

Befindet sich der Spritzenkolben 9 an seiner bestimmungsgemäßen Position, wird das Betätigungselement 3' des Klemmmittels 3 losgelassen.

Die Feder 7 entspannt sich und drückt die Druckplatte 8 nach unten auf den hakenähnlichen Vorsprung 12. Dadurch dreht sich die Querstrebe 11 zurück und bewegt sich gleichzeitig axial zur Spritze im Langloch 15 nach unten, d.h. weg vom Spritzenkolben 9. Der kurvenähnliche Vorsprung 13 gleitet von der Erhebung 16 zurück in die Vertiefung 14.

Durch diese gleichzeitig stattfindende translatorische und rotatorische Bewegung ergreift das Haltelement den Kolbenflansch von der Seite und von oben einschenkend. Das klammerähnliche Ende 4 des Haltelements 3" kommt auf der inneren Seite des Kolbenflansches 10 zum Liegen.

Somit ist eine spielfreie, feste, sichere und exakt positionierte Halterung des Spritzenkolbens auch bei variierenden Kolbenflanschstärken von 1,2 mm bis 3,00 mm gewährleistet.

Um das Haltelement 3" wieder vom Kolbenflansch 10 zu trennen, wird das Betätigungselement erneut betätigt. So ist ein komfortables Entnehmen der Spritze möglich.

Eine geeignete Kombination von Vorspannung der Feder und Nockenkurve ermöglicht die Einhandbenutzung der Aufnahmevorrichtung.

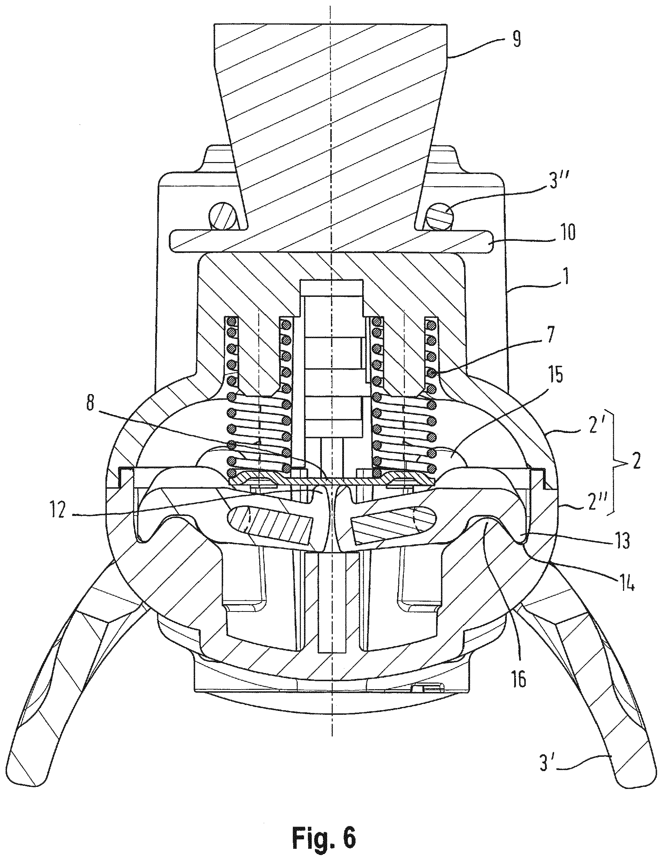

**Figur 6** zeigt in schematischer Darstellung eine Ausführungsform der erfindungsgemäßen Aufnahmevorrichtung 1 mit geschlossenen Klemmmitteln im Längsschnitt.

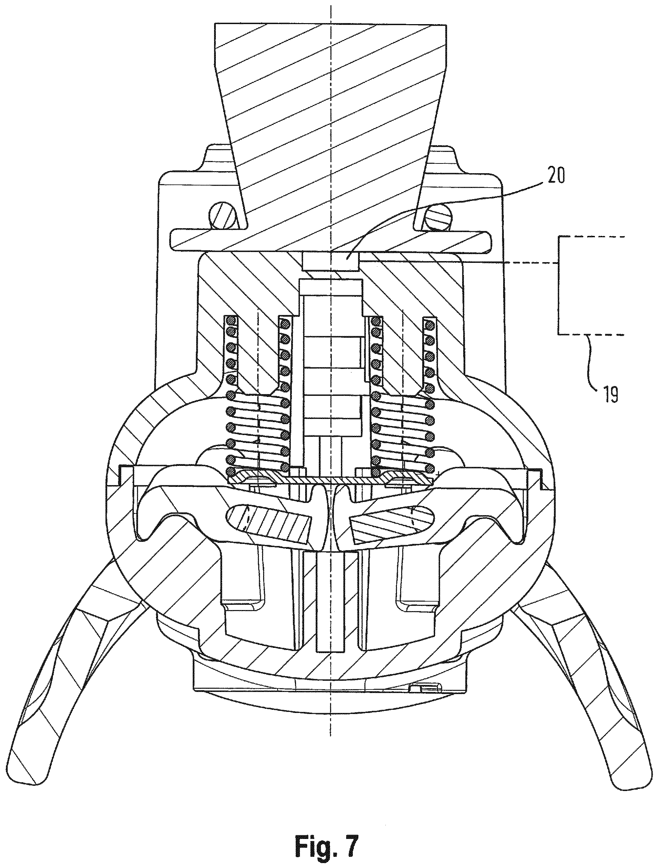

**Figur 7** zeigt in schematischer Darstellung eine Ausführungsform der erfindungsgemäßen Aufnahmevorrichtung 1 mit geschlossenen Klemmmitteln im Längsschnitt mit einer Auswerteeinheit 19, die mit mindestens einem Sensor 20 verbunden ist. Der Sensor 20 befindet sich vorzugsweise im oberen Teil des Gehäuses 2 und lässt Aussagen über die Position des Kolbenflansches 9 und der Halteelemente 3" zu. Ist die Spritze nicht korrekt eingelegt, d.h. liegt der Kolbenflansch 9 nicht plan an der Aufnahmefläche 2"' des Gehäuses 2 an, oder haben die Haltelemente sich nicht bestimmungsgemäß geöffnet oder geschlossen, wird ein Alarmsignal produziert oder der Betrieb der Dosiervorrichtung gehemmt.

| | |
|---|---|
| 1 | Halterung |
| 2 | Gehäuse |
| 2' | oberer Teil des Gehäuses |
| 2" | unterer Teil des Gehäuses |
| 3 | Klemmmittel |
| 3' | Betätigungselement des Klemmmittels |
| 3" | Halteelement des Klemmmittels |
| 4 | klammerähnliches Ende des Halteelements |
| 5 | Öffnung für Gleitstange |
| 6 | Kolbenflanschauflage |
| 7 | Feder |
| 8 | Druckplatte |
| 9 | Spritzenkolben |
| 10 | Kolbenflansch |
| 11 | Querstrebe |
| 12 | hakenähnlicher Vorsprung |
| 13 | kurvenähnlicher Vorsprung |
| 14 | Vertiefung |
| 15 | Langloch |
| 16 | Erhebung |
| 17 | Abstandsrad |
| 18 | Aufnahmevorrichtung |
| 19 | Auswerteeinheit |
| 20 | Sensor |
| 21 | Fluidabgabevorrichtung |
| 21' | Gleitstange |
| 21" | Spritzenhalterung |
| 21"' | Befestigungsvorrichtung |

## Patentansprüche

1. Vorrichtung zur Aufnahme einer Spritze in eine Fluidabgabevorrichtung (21) mit mindestens zwei Klemmmitteln (3), die federnd vorspannbar sind, wobei die Klemmmittel den Kolbenflansch (10) eines Spritzenkolbens (9) greifen, **dadurch gekennzeichnet, dass**
• das Klemmmittel (3) aus einem Halteelement (3") und einem Betätigungsmittel (3') besteht,
• das Klemmmittel (3) eine Querstrebe (11) besitzt,
• an der Querstrebe (11) des Klemmmittels (3) mindestens zwei Vorsprünge (12, 13) ausgebildet sind und
• das Klemmmittel (3) mittels der Querstrebe (11) in einem Gehäuse (2) gehaltert ist, wobei das Klemmmittel (3) vorgespannt ist durch mindestens eine in dem Gehäuse (2) gelagerte Feder (7), die über eine Druckplatte (8) in Verbindung mit einem der Vorsprünge (12, 13) der Querstrebe (11) steht, und wobei in dem Gehäuse (2) für jedes Klemmmittel (3) ein Langloch (15), in dem die Querstrebe axial verschiebbar und um die eigene Achse drehbar angeordnet ist, mindestens eine Erhebung (16) und mindestens eine Vertiefung (14) ausgebildet sind, welche mit der Querstrebe (11) und deren Vorsprüngen (12, 13) korrespondierend in Verbindung stehen.

2. Vorrichtung nach Anspruch 1, **dadurch gekennzeichnet, dass** das Halteelement (3") und das Betätigungsmittel (3') des Klemmmittels (3) einstückig ausgebildet sind.

3. Vorrichtung nach Anspruch 1, **dadurch gekennzeichnet, dass** einer der an der Querstrebe (11) befindlichen Vorsprünge hakenähnlich (12) und ein anderer der an der Querstrebe (11) befindlichen Vorsprünge kurvenähnlich (13) ausgebildet ist.

4. Vorrichtung nach Anspruch 1 und 3, **dadurch gekennzeichnet, dass** der hakenähnliche Vorsprung (12) der Querstrebe (11) mit der Druckplatte (8) in Verbindung steht und der kurvenähnliche Vorsprung (13) der Querstrebe (11) Kontakt mit der Vertiefung (14) und der Erhebung (16) des Gehäuses (2) aufnimmt.

5. Vorrichtung nach Anspruch 1, **dadurch gekennzeichnet, dass** das Gehäuse (2) aus einer oberen Hälfte (2') und einer unteren Hälfte (2") besteht.

6. Vorrichtung nach einem der vorhergehenden Ansprüche, **dadurch gekennzeichnet, dass** die Fluidabgabevorrichtung (21) eine Dosiervorrichtung, vorzugsweise eine Dosierpumpe und insbesondere eine Spritzenpumpe ist.

7. Vorrichtung nach einem der vorhergehenden Ansprüche, **dadurch gekennzeichnet, dass** die Fluidabgabevorrichtung (21) eine Heparinpumpe ist.

8. Vorrichtung nach einem der vorhergehenden Ansprüche, **dadurch gekennzeichnet, dass** sie aus einem formstabilen Material, vorzugsweise Kunststoff oder Metall besteht.

9. Vorrichtung nach einem der vorhergehenden Ansprüche, **dadurch gekennzeichnet, dass** der aufzunehmende Kolbenflansch (10) in einer Stärke von 1,2 mm bis 3 mm variiert.

10. Vorrichtung nach einem der vorhergehenden Ansprüche, **dadurch gekennzeichnet, dass** der Winkel der Erhebung (16) zwischen 0 und 50° beträgt.

11. Vorrichtung nach einem der vorhergehenden Ansprüche, **dadurch gekennzeichnet, dass** sie einhändig bedienbar ist.

12. Verfahren zur Aufnahme einer Spritze in eine Fluidabgabevorrichtung (21) gemäß Anspruch 1, **dadurch gekennzeichnet, dass** das Greifen des Kolbenflansches (10) eines Spritzenkolbens (9) durch die Klemmmittel (3) und das Loslassen derselben eine axial translatorische und eine rotatorische Bewegung darstellt.

13. Verfahren nach Anspruch 12, **dadurch gekennzeichnet, dass** die translatorische und die rotatorische Bewegung zeitgleich ausgeführt werden.

14. Verfahren nach Anspruch 12 und 13, **dadurch gekennzeichnet, dass** der Greifvorgang bei der Aufnahme des Kolbenflansches (10) des Spritzenkolbens (9) folgende Schritte umfasst
• Aktivierung des Betätigungsmittels (3') zur Abspreizung des Halteelements (3"), wobei
- der kurvenähnliche Vorsprung (13) der Querstrebe (11) des Betätigungsmittels (3') aus der Vertiefung (14) auf die Erhebung (16) gleitet (rotatorische Bewegung),
- die Querstrebe (11) sich im Langloch (15) axial zum Spritzenkolben (9) von distal nach proximal bewegt (translatorische Bewegung) und
- die Feder (7) durch den hakenähnlichen Vorsprung (12) über die Druckplatte (8) zusammengepresst wird,
• Loslassen des Betätigungsmittels (3') zum Greifen des Kolbenflansches (10) durch das Halteelement (3"), wobei
- der kurvenähnliche Vorsprung (13) der Querstrebe (11) des Betätigungsmittels (3') von der Erhebung (16) in die Vertiefung (14) zurückgleitet (rotatorische Bewegung),
- die Querstrebe (11) sich im Langloch (15) axial zum Spritzenkolben (9) von proximal nach distal bewegt (translatorische Bewegung) und
- der hakenähnliche Vorsprung (12) die Druckplatte (8) absinken lässt, wobei sich die Federn (7) ausdehnen und die Klemmmittel (3) vorspannen.

15. Medizintechnische Behandlungseinheit mit einer Vorrichtung nach Anspruch 1.

16. Auswerteeinheit (19) für eine Vorrichtung gemäß Anspruch 1 mit mindestens einem Sensor (20), über welchen eine Aussage über die Position des Spritzenkolbens (9) und/oder des Halteelements (3") zu treffen ist.

## Claims

1. A device for holding a syringe in a fluid-dispensing device (21) having at least two clamping means (3) which can be biased resiliently, wherein the clamping means grip the plunger flange (10) of a syringe plunger (9),
**characterized in that**
• the clamping means (3) consists of a holding element (3") and an actuating element (3'),
• the clamping means (3) has a transverse strut (11),
• at least two protrusions (12, 13) are formed on the transverse strut (11) of the clamping means (3), and
• the clamping means (3) is held in a housing (2) by means of the transverse strut (11), wherein the clamping means (3) is prestressed by at least one spring (7) mounted in the housing (2) and connected to one of the protrusions (12, 13) of the transverse strut (11) by means of a pressure plate (8), and wherein an elongated hole (15), in which the transverse strut is axially displaceable and is arranged to be rotatable about its own axis, is provided for each clamping means (3) in the housing (2), at least one elevation (16) and at least one recess (14) being formed, communicating with the transverse strut (11) and its protrusions (12, 13) accordingly.

2. The device according to claim 1,
**characterized in that**
the holding element (3") and the actuating element (3') of the clamping means (3) are designed in one piece.

3. The device according to claim 1,
**characterized in that**
one of the protrusions on the transverse strut (11) is designed like a hook (12), and the other of the protrusions situated on the transverse strut (11) is designed like a curve (13).

4. The device according to claims 1 and 3,
**characterized in that**
the hook-like protrusion (12) on the transverse strut (11) is connected to the pressure plate (8) and the curve-like protrusion (13) on the transverse strut (11) comes in contact with the recess (14) and an elevation (16) of the housing (2).

5. The device according to claim 1,
**characterized in that**
the housing (2) consists of an upper half (2') and a lower half (2'').

6. The device according to any one of the preceding claims,
**characterized in that**
the fluid-dispensing device (21) is a dosing device, preferably a dosing pump, and in particular syringe pump.

7. The device according to any one of the preceding claims,
**characterized in that**
the fluid-dispensing device (21) is a heparin pump.

8. The device according to any one of the preceding claims,
**characterized in that**
it is made of a material having dimensional stability, preferably made of plastic or metal.

9. The device according to any one of the preceding claims,
**characterized in that**
the plunger flange (10) to be accommodated is varied in a thickness of 1.2 mm to 3 mm.

10. The device according to any one of the preceding claims,
**characterized in that**
the angle of the elevation (16) is between 0° and 50°.

11. The device according to any one of the preceding claims,
**characterized in that**
it can be operated with one hand.

12. A method for holding a syringe in a fluid-dispensing device (21) according to claim 1,
**characterized in that**
the gripping and the release of the plunger flange (10) of a syringe plunger (9) by the clamping means (3), constitutes an axially translational movement and a rotational movement.

13. The method according to claim 12,
**characterized in that**
the translational and rotational movements are carried out at the same time.

14. The method according to claims 12 and 13,
**characterized in that**
the gripping operation in holding the plunger flange (10) of the syringe plunger (9) comprises the following steps:
• activation of the actuating means (3') for spreading the holding element (3"), wherein
- the curve-like protrusion (13) on the transverse strut (11) of the actuating means (3') slides out of the recess (14) onto the elevation (16) (rotational movement),
- the transverse strut (11) is moved axially from distal to proximal in the elongated hole (15) relative to the syringe plunger (9) (translational movement), and
- the spring (7) is compressed by the hook-like protrusion (12) via the pressure plate (8),
• releasing the actuating means (3') for gripping the plunger flange (10) by the holding element (3''), wherein
- the curve-like protrusion (13) of the transverse strut (11) of the actuating means (3') slides back from the elevation (16) into the recess (14) (rotational movement),
- the transverse strut (11) moves axially from proximal to distal in the elongated hole (15) relative to the syringe plunger (9) (translational movement), and
- the hook-like protrusion (12) causes the pressure plate (8) to sink, wherein the springs (7) expand, and put the clamping means (3) under a prestress.

15. A technical medical unit having a device according to claim 1.

16. An evaluation unit (19) for a device according to claim 1, comprising at least one sensor (20) by means of which a statement can be made about the position of the syringe plunger (9) and/or of the holding element (3'').

## Revendications

1. Dispositif destiné à recevoir une seringue dans un dispositif distributeur de fluide (21), comportant au moins deux moyens de serrage (3) qui peuvent être précontraints élastiquement, les moyens de serrage entourant la bride de piston (10) d'un piston de seringue (9), **caractérisé en ce que**
• le moyen de serrage (3) est composé d'un élément de retenue (3'') et d'un moyen d'actionnement (3'),
• le moyen de serrage (3) comporte une traverse (11),
• au niveau de la traverse (11) du moyen de serrage (3), au moins deux saillies (12, 13) sont constituées et
• le moyen de serrage (3) s'appuie au moyen de la traverse (11) dans un boîtier (2), le moyen de serrage (3) étant précontraint par au moins un ressort (7) s'appuyant dans le boîtier (2) et qui est en liaison par une plaque de compression (8) avec une des saillies (12, 13) de la traverse (11), et étant pratiqué dans le boîtier (2) pour chaque moyen de serrage (3) un trou oblong (15) dans lequel la traverse est déplaçable axialement et étant réalisées, disposées de manière à pouvoir tourner autour de leur axe, au moins une protubérance (16) et au moins un creux (14) qui sont en liaison de manière correspondante avec la traverse (11) et ses saillies (12, 13).

2. Dispositif selon la revendication 1, **caractérisé en ce que** l'élément de retenue (3'') et l'élément d'actionnement (3') du moyen de serrage (3) sont monobloc.

3. Dispositif selon la revendication 1, **caractérisé en ce qu'**une des saillies se trouvant au niveau de la traverse (11) est réalisée en forme de crochet (12) et qu'une autre des saillies se trouvant au niveau de la traverse (11) est réalisée en forme de courbe (13).

4. Dispositif selon la revendication 1 et 3, **caractérisé en ce que** la saillie en forme de crochet (12) de la traverse (11) est en liaison avec la plaque de compression (8) et que la saillie en forme de courbe (13) de la traverse (11) vient en contact avec le creux (14) et la protubérance (16) du boîtier (2).

5. Dispositif selon la revendication 1, **caractérisé en ce que** le boîtier (2) est composé d'une moitié supérieure (2') et d'une moitié inférieure (2'').

6. Dispositif selon une des revendications précédentes, **caractérisé en ce que** le dispositif distributeur de fluide (21) est un dispositif de dosage, de préférence une pompe de dosage et en particulier une pompe d'injection.

7. Dispositif selon une des revendications précédentes, **caractérisé en ce que** le dispositif distributeur de fluide (21) est une pompe à héparine.

8. Dispositif selon une des revendications précédentes, **caractérisé en ce qu'**il est composé d'un matériau de forme stable, de préférence du plastique ou du métal.

9. Dispositif selon une des revendications précédentes, **caractérisé en ce que** la bride de piston à recevoir (10) a une épaisseur qui varie de 1,2 mm à 3 mm.

10. Dispositif selon une des revendications précédentes, **caractérisé en ce que** l'angle de la protubérance (16) est compris entre 0 et 50°.

11. Dispositif selon une des revendications précédentes, **caractérisé en ce qu'**il est maniable d'une seule main.

12. Procédé de réception d'une seringue dans un dispositif distributeur de fluide (21) selon la revendication 1, **caractérisé en ce que** la saisie de la bride de piston (10) d'un piston d'injection (9) par le moyen de serrage (3) et le relâchement de celui-ci constituent un mouvement axialement translatoire et rotatif.

13. Procédé selon la revendication 12, **caractérisé en ce que** le mouvement translatoire et le mouvement rotatif sont effectués simultanément.

14. Procédé selon les revendications 12 et 13, **caractérisé en ce que** l'opération de saisie lors de la réception de la bride de piston (10) du piston d'injection (9) comprend les étapes suivantes
• activation du moyen d'actionnement (3') pour écarter l'élément de retenue (3''),
- la saillie en forme de courbe (13) de la traverse (11) de l'élément d'actionnement (3') glissant hors du creux (14) sur la protubérance (16) (mouvement rotatoire),
- la traverse (11) se déplaçant dans le trou oblong (15) axialement par rapport au piston d'injection (9) dans le sens distal à proximal (mouvement translatoire) et
- le ressort (7) étant comprimé par la saillie en forme de crochet (12) par l'intermédiaire de la plaque compression (8),
• relâchement du moyen d'actionnement (3') pour la saisie de la bride de piston (10) par l'élément de retenue (3''),
- la saillie en forme de courbe (13) de la traverse (11) de l'élément d'actionnement (3') retournant en glissant de la protubérance (16) vers l'intérieur du creux (14) (mouvement rotatoire),
- la traverse (11) se déplaçant dans le trou oblong (15) axialement par rapport piston d'injection (9) dans le sens proximal à distal (mouvement translatoire) et
- la saillie en forme de crochet (12) faisant descendre la plaque de compression (8), les ressorts (7) s'étirant et précontraignant les moyens de serrage (3).

15. Unité technique de traitement médical comportant un dispositif selon la revendication 1.

16. Unité d'exploitation (19) pour dispositif selon la revendication 1, comportant au moins capteur (20), qui permet de faire une détermination de la position du piston d'injection (9) et/ou de l'élément de retenue (3'').
